# EUROPEAN PATENT SPECIFICATION

(11) **EP 4 443 140 B1**
(45) Date of publication and mention of the grant of the patent: **18.03.2026**
(21) Application number: 24167723.6
(22) Date of filing: 28.03.2024
(51) Int. Cl.: G01N 21/85, B64G 1/60, C02F 1/00

(54) **OPTICAL SENSOR**
OPTISCHER SENSOR
CAPTEUR OPTIQUE

(30) Priority: 07.04.2023 US 202318297091
(43) Date of publication of application: 09.10.2024
(73) Proprietor: Hamilton Sundstrand Space Systems International, Inc., Charlotte, NC 28217 (US)
(72) Inventor: MYERS, Casey, Parker, CO (US); KAUFMAN, Cory, St. Louis, MO (US)
(74) Representative: Dehns

(56) References cited:
- WO-A1-2018/071539
- US-A1- 2016 054 281
- US-B2- 10 859 070

## Description

### BACKGROUND

Exemplary embodiments pertain to the art of fluid systems, and in particular to measurement of properties of the fluid in the system.

In fluid systems, such as waste treatment systems, it is desired to measure properties of the fluid, such as concentrations of additives in the fluid or mix quality or homogeneity of the mixture of the fluid. Typically, such determinations are made by using one or more conductivity sensors. Conductivity sensors, however, are electrode-based conductivity sensors that require that critical sensing elements are immersed in corrosive or potentially incompatible fluids, which decreases reliability of the sensors and shortens their useful service life. Alternatively, inductive fluid sensors require high ion levels in the fluid to sense due to the non-contact nature of inductance and the need to generate (induce) a magnetic field. A waste processing system and method are known from US 2016/206160.

### BRIEF DESCRIPTION

In one embodiment, a waste processing system includes a storage tank to collect urine, a dose pump to mix a volume of urine pretreat with a volume of water and dispense the resultant pretreat mixture into the storage tank, and a sensor configured to monitor one or more properties of the pretreat mixture. The sensor is an optical sensor transmitting a light signal through the pretreat mixture.

Additionally or alternatively, in this or other embodiments a light source of the optical sensor is a light emitting diode (LED).

Additionally or alternatively, in this or other embodiments the one or more of the wavelength, frequency, or intensity of the light signal can be varied.

Additionally or alternatively, in this or other embodiments the light signal is transmitted through opposing optical windows in a fluid pathway through which the pretreat mixture is flowed.

Additionally or alternatively, in this or other embodiments the light signal is received at a photodiode after passing through the pretreat mixture.

Additionally or alternatively, in this or other embodiments an optical filter is located between the light source and the pretreat mixture.

Additionally or alternatively, in this or other embodiments the one or more properties include a concentration of urine pretreat in the pretreat mixture and a mixture quality of the pretreat mixture.

Additionally or alternatively, in this or other embodiments a controller compares the one or more properties to a preselected range.

In another embodiment, a method of monitoring a flow of a pretreat mixture of a waste processing system includes mixing a volume of urine pretreat with a volume of water at a dose pump defining a pretreat mixture, directing the pretreat mixture along a fluid pathway toward a storage tank, and measuring one or more properties of the pretreat mixture via an optical sensor disposed along the fluid pathway.

Additionally or alternatively, in this or other embodiments the optical sensor transmits a light signal through the fluid pathway via a light source, and receives the light signal at a photodiode after passing through the pretreat mixture.

Additionally or alternatively, in this or other embodiments the light source is an LED light source.

Additionally or alternatively, in this or other embodiments one or more of a wavelength, frequency, or intensity of the light signal is varied.

Additionally or alternatively, in this or other embodiments the light signal is transmitted through opposing optical windows in the fluid pathway.

Additionally or alternatively, in this or other embodiments the light signal is transmitted through a filter located between the light source and the fluid pathway.

Additionally or alternatively, in this or other embodiments the one or more properties is compared to a preselected range.

Additionally or alternatively, in this or other embodiments an alarm is initiated when the one or more properties are outside of the preselected range.

Additionally or alternatively, in this or other embodiments the one or more properties include a concentration of urine pretreat in the pretreat mixture and a mixture quality of the pretreat mixture.

### BRIEF DESCRIPTION OF THE DRAWINGS

The following descriptions should not be considered limiting in any way. With reference to the accompanying drawings, like elements are numbered alike:
FIG. 1 is a partial schematic illustration of an embodiment of a waste processing system;
FIG. 2 is a schematic illustration of an embodiment of an optical pretreat quality sensor; and
FIG. 3 is a schematic illustration of another embodiment of a waste processing system;
FIG. 4 is an illustration of an embodiment of a method of monitoring a pretreat mixture 28.

### DETAILED DESCRIPTION

A detailed description of one or more embodiments of the disclosed apparatus and method are presented herein by way of exemplification and not limitation with reference to the Figures.

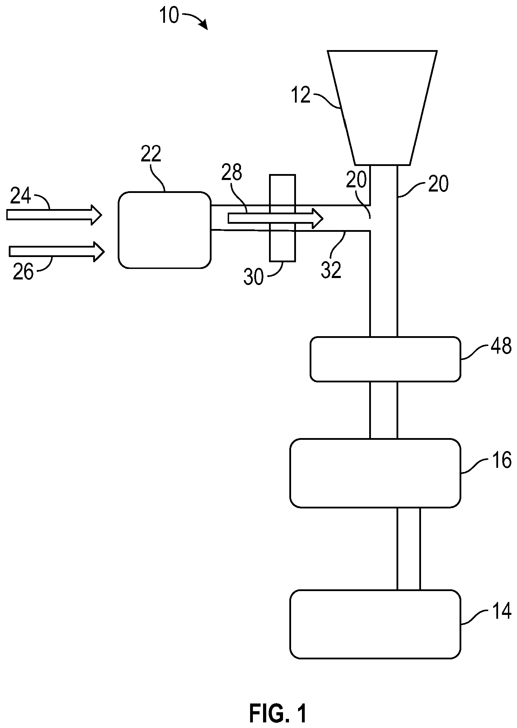

Referring now to FIG. 1, illustrated is a schematic of a fluid system, in this embodiment a waste processing system 10 of an extraplanetary habitat, such as a space station, extraplanetary base, or other microgravity environment. The waste processing system 10 processes waste and includes components to reclaim water from urine. Urine is collected at a toilet 12 and is conveyed to a urine processing assembly (UPA) 14, which in some embodiments utilizes a vacuum distillation process that removes most of the organics and solids from the urine. The UPA 14 operates in batches, so prior to being processed by the UPA 14, the urine is stored at a storage tank 16. Because of this storage and batch processing, the urine must be stabilized upon each use of the toilet to inhibit molecular breakdown, organic growth, and/or solids precipitation. To stabilize the urine, it is mixed with a phosphoric acid with chromate during each use of the toilet 12. In some embodiments, this additive, referred to as urine pretreat, is input into the urine stream in the waste processing system 10 at, for example, an additive inlet 20. In some embodiments, a dose pump 22 mixes a concentration of the urine pretreat, shown as 24, with a volume of water 26 to dilute the urine pretreat 24 into a pretreat mixture 28 before adding it to the urine stream at the additive inlet 20.

It is desired to know the ratio of urine pretreat 24 to water 26 in the pretreat mixture 28 exiting the dose pump 22 to ensure proper functioning of the dose pump 22 and thus proper stabilization of the urine. A pretreat quality sensor 30 is located in a mixture pathway 32 between the dose pump 22 and the additive inlet 20 to measure the desired properties of the pretreat mixture 28, such as concentration of the urine pretreat 24 in the pretreat mixture 28 as well as the homogeneity, or mix quality, of the pretreat mixture 28. The measured values may be compared to target values to evaluate the performance of the UPA 14. To perform these measurements, the pretreat quality sensor 30 is an optical pretreat quality sensor 30. While in the illustrated embodiments, the sensor 30 is utilized to measure concentration of the urine pretreat 24 in the pretreat mixture 28, one skilled in the art will readily appreciate that the sensor 30 may be utilized to monitor two-phase flow, for example, gas or solids in a liquid stream. Further, in some embodiments the waste processing system 10 may include a phase separator 48 to separate gas and liquid fluids before entering the storage tank 16, In some embodiments, the phase separator 48 is located downstream of the additive inlet 20. Further, as shown in the illustrated embodiment, the additive inlet 20 is located upstream of the storage tank 16 along the mixture pathway 32, while in other embodiments the additive inlet 20 may deposit the pretreat mixture 28 directly into the storage tank 16 for mixing with the urine therein.

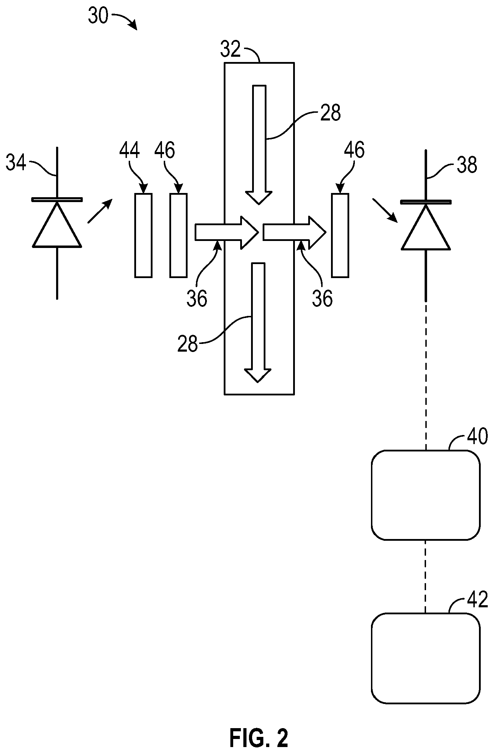

Referring now to FIG. 2, The optical pretreat quality sensor 30 includes a light source 34, such as an LED light source 34, that emits a light signal 36 through the pretreat mixture 28 and toward a light receiving device, such as a photodiode 38. The light signal 36 is received at the photodiode 38 and evaluated by a controller 40, where the measured properties, based on the received light signal 36, are compared to a selected value or range of values. If the properties are outside of the selected range, the controller 40 may initiate an audial and/or visual alarm 42 to alert the users of the condition.

As stated above, in some embodiments the light source 34 is an LED light source, but one skilled in the art will readily appreciate that other light sources may be used. The light source 34 may be a variable frequency, wavelength, or intensity light source 34, so that the light signal 36 may be varied based on the fluid properties to be evaluated and/or the type of fluid to be evaluated. In some embodiments, the light signal 36 emitted by the light source 34 is passed through a filter 44 to change, for example, or isolate a wavelength of the light signal 36. The optical pretreat quality sensor 30 does not come in contact with the pretreat mixture 28 in the mixture pathway 32. Instead, optical windows 46 are located in the mixture pathway 32, through which the light signal 36 is transmitted across the mixture pathway 32 and through the pretreat mixture 28.

In some embodiments, the fluid pathway 32 is a glass or optically clear tube. In such embodiments, the optical windows 46 may be eliminated. Eliminating the optical windows 46 reduces the number of corners or crevices along the fluid pathway 32 in which gas may get trapped, thus increasing an accuracy and reliability of the data received from the optical pretreat quality sensor 30. The optical pretreat quality sensor 30 may or may not be mechanically connected to the fluid pathway 32. In some embodiments, the optical pretreat quality sensor 30 is a hand-held portable device.

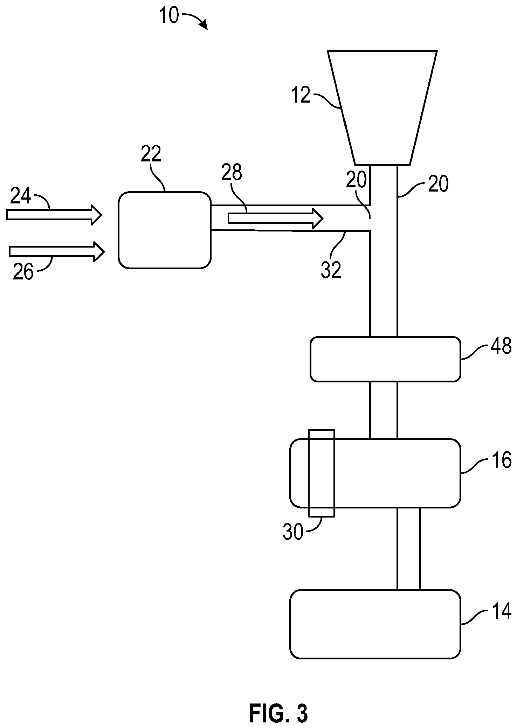

While in the embodiment of FIG. 1, the optical pretreat quality sensor 30 is located upstream of the storage tank, in other embodiments, such as in FIG. 3, the optical pretreat quality sensor 30 is alternatively or additionally located at the storage tank 16 to monitor the conditions within the storage tank 16.

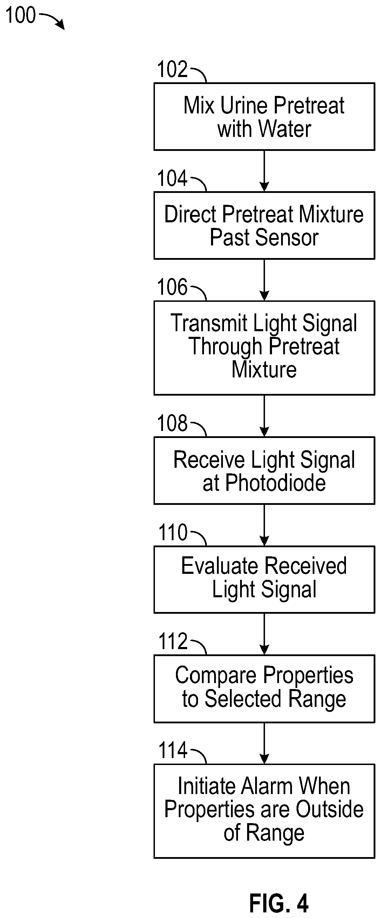

Referring now to FIG. 4, a method 100 of monitoring flow of a pretreat mixture 28 will now be described. At step 102, urine pretreat 24 is mixed with water 26 at the dose pump 22. At step 104, the resultant pretreat mixture 28 is passed through the mixture pathway 32 past the optical pretreat quality sensor 30. At step 106 the light source 34 transmits the light signal 36 through the mixture pathway 32. The light signal 36 is received at the photodiode 38 at step 108, and the light signal 36 is evaluated by the controller 40 to determine one or more properties of the pretreat mixture 28 at step 110. At step 112, the measured properties may be compared to a selected range, and at step 114 the controller 40 may initiate an alarm if the measured properties are outside of the selected range.

Use of the optical pretreat quality sensor 30 keeps the sensing elements out of contact with the pretreat mixture 28, thus reducing contamination or corrosion of the sensing elements, thereby extending their service life. This further ensures continued accuracy of the measurements of the properties of the pretreat mixture 28. While in the present disclosure the fluid being evaluated is a pretreat mixture 28, one skilled in the art will readily appreciate that the sensor 30 arrangement disclosed herein may be utilized to evaluate properties of other fluids and mixtures, such as water quality, urine, fuel, coolant, 2 phase flow, liquid & gas for example, or the like. Additionally, the optical sensor 30 may be utilized to measure characteristics or composition of the urine as a monitor of crew members' health, or to measure characteristics of fluids such as a potable water supply aboard, for example, an extraplanetary vehicle or at an extraplanetary habitat. Further, the optical sensor 30 may be utilized to determine ratios and/or phases of fluids in other systems, such as environmental control and life support systems of an extraplanetary vehicle or habitat.

The term "about" is intended to include the degree of error associated with measurement of the particular quantity based upon the equipment available at the time of filing the application.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. **In** addition, many modifications may be made to adapt a particular situation or material to the teachings of the present disclosure without departing from the essential scope thereof. Therefore, it is intended that the present disclosure not be limited to the particular embodiment disclosed as the best mode contemplated for carrying out this present disclosure, but that the present disclosure will include all embodiments falling within the scope of the claims.

## Claims

1. A waste processing system, comprising:
a storage tank (16) to collect urine;
a dose pump (22) to mix a volume of urine pretreat with a volume of water and dispense the resultant pretreat mixture into the storage tank (16); and
a sensor (30) configured to monitor one or more properties of the pretreat mixture;
wherein the sensor (30) is an optical sensor transmitting a light signal through the pretreat mixture.

2. The waste processing system of claim 1, wherein a light source (34) of the optical sensor (30) is a light emitting diode (LED).

3. The waste processing system of claim 1 or 2, wherein the one or more of the wavelength, frequency, or intensity of the light signal can be varied.

4. The waste processing system of any preceding claim, wherein the light signal is transmitted through opposing optical windows (46) in a fluid pathway through which the pretreat mixture is flowed.

5. The waste processing system of any preceding claim, wherein the light signal is received at a photodiode (38) after passing through the pretreat mixture.

6. The waste processing system of any preceding claim, further comprising an optical filter disposed between the light source and the pretreat mixture.

7. The waste processing system of any preceding claim, wherein the one or more properties include a concentration of urine pretreat in the pretreat mixture and a mixture quality of the pretreat mixture.

8. The waste processing system of any preceding claim, further comprising a controller (40) to compare the one or more properties to a preselected range.

9. A method of monitoring a flow of a pretreat mixture of a waste processing system, comprising:
mixing a volume of urine pretreat with a volume of water at a dose pump (22) defining a pretreat mixture;
directing the pretreat mixture along a fluid pathway toward a storage tank (16);
measuring one or more properties of the pretreat mixture via an optical sensor (30) disposed along the fluid pathway.

10. The method of claim 9, wherein the optical sensor:
transmits a light signal through the fluid pathway via a light source (34), and
receives the light signal at a photodiode (38) after passing through the pretreat mixture.

11. The method of claim 10, wherein the light source is an LED light source; and/or further comprising varying one or more of a wavelength, frequency, or intensity of the light signal; and/or further comprising transmitting the light signal through opposing optical windows (46) in the fluid pathway.

12. The method of claim 10 or 11, further comprising transmitting the light signal through a filter disposed between the light source and the fluid pathway.

13. The method of any of claims 9-12, further comprising comparing the one or more properties to a preselected range.

14. The method of claim 13, further comprising initiating an alarm when the one or more properties are outside of the preselected range.

15. The method of any of claims 9-14, wherein the one or more properties include a concentration of urine pretreat in the pretreat mixture and a mixture quality of the pretreat mixture.

## Patentansprüche

1. Abfallverarbeitungssystem, umfassend:
einen Sammelbehälter (16) zum Sammeln von Urin;
eine Dosierpumpe (22) zum Mischen einer Menge Urinvorbehandlung mit einer Menge Wasser und zum Abgeben der resultierenden Vorbehandlungsmischung in den Sammelbehälter (16); und
einen Sensor (30), der dazu ausgelegt ist, eine oder mehrere Eigenschaften der Vorbehandlungsmischung zu überwachen;
wobei der Sensor (30) ein optischer Sensor ist, der ein Lichtsignal durch die Vorbehandlungsmischung überträgt.

2. Abfallverarbeitungssystem nach Anspruch 1, wobei die Lichtquelle (34) des optischen Sensors (30) eine Leuchtdiode (LED) ist.

3. Abfallverarbeitungssystem nach Anspruch 1 oder 2, wobei das eine oder die mehreren der Wellenlänge, der Frequenz oder der Intensität des Lichtsignals geändert werden können.

4. Abfallverarbeitungssystem nach einem der vorhergehenden Ansprüche, wobei das Lichtsignal durch gegenüberliegende optische Fenster (46) in einem Fluidweg übertragen wird, durch den die Vorbehandlungsmischung fließt.

5. Abfallverarbeitungssystem nach einem der vorhergehenden Ansprüche, wobei das Lichtsignal nach dem Durchtreten durch die Vorbehandlungsmischung an einer Fotodiode (38) empfangen wird.

6. Abfallverarbeitungssystem nach einem der vorhergehenden Ansprüche, ferner umfassend ein optisches Filter, das zwischen der Lichtquelle und der Vorbehandlungsmischung angeordnet ist.

7. Abfallverarbeitungssystem nach einem der vorhergehenden Ansprüche, wobei die eine oder die mehreren Eigenschaften eine Konzentration der Urinvorbehandlung in der Vorbehandlungsmischung und eine Mischungsqualität der Vorbehandlungsmischung beinhalten.

8. Abfallverarbeitungssystem nach einem der vorhergehenden Ansprüche, ferner umfassend eine Steuereinheit (40) zum Vergleichen der einen oder der mehreren Eigenschaften mit einem vorab ausgewählten Bereich.

9. Verfahren zur Überwachung eines Durchflusses einer Vorbehandlungsmischung eines Abfallverarbeitungssystems, umfassend:
Mischen einer Menge Urinvorbehandlung mit einer Menge Wasser an einer Dosierpumpe (22), um eine Vorbehandlungsmischung zu definieren;
Leiten der Vorbehandlungsmischung über einen Fluidweg zu einem Sammelbehälter (16);
Messen einer oder mehrerer Eigenschaften der Vorbehandlungsmischung über einen optischen Sensor (30), der entlang des Fluidwegs angeordnet ist.

10. Verfahren nach Anspruch 9, wobei der optische Sensor:
ein Lichtsignal über eine Lichtquelle (34) durch den Fluidweg überträgt und
das Lichtsignal nach dem Durchtreten durch die Vorbehandlungsmischung an einer Fotodiode (38) empfängt.

11. Verfahren nach Anspruch 10, wobei die Lichtquelle eine LED-Lichtquelle ist; und/oder ferner umfassend Ändern eines oder mehrerer von einer Wellenlänge, einer Frequenz oder einer Intensität des Lichtsignals; und/oder ferner umfassend Übertragen des Lichtsignals durch gegenüberliegende optische Fenster (46) in dem Fluidweg.

12. Verfahren nach Anspruch 10 oder 11, ferner umfassend Übertragen des Lichtsignals durch ein Filter, das zwischen der Lichtquelle und dem Fluidweg angeordnet ist.

13. Verfahren nach einem der Ansprüche 9-12, ferner umfassend Vergleichen der einen oder der mehreren Eigenschaften mit einem vorab ausgewählten Bereich.

14. Verfahren nach Anspruch 13, ferner umfassend Auslösen eines Alarms, wenn die eine oder die mehreren Eigenschaften außerhalb des vorab ausgewählten Bereichs liegen.

15. Verfahren nach einem der Ansprüche 9-14, wobei die eine oder die mehreren Eigenschaften eine Konzentration der Urinvorbehandlung in der Vorbehandlungsmischung und eine Mischungsqualität der Vorbehandlungsmischung beinhalten.

## Revendications

1. Système de traitement des déchets, comprenant ;
un réservoir de stockage (16) pour recueillir l'urine ;
une pompe doseuse (22) pour mélanger un volume de prétraitement urinaire avec un volume d'eau et distribuer le mélange de prétraitement résultant dans le réservoir de stockage (16) ; et un capteur (30) configuré pour surveiller une ou plusieurs propriétés du mélange de prétraitement ;
dans lequel le capteur (30) est un capteur optique transmettant un signal lumineux à travers le mélange de prétraitement.

2. Système de traitement des déchets selon la revendication 1, dans lequel une source lumineuse (34) du capteur optique (30) est une diode électroluminescente (DEL).

3. Système de traitement des déchets selon la revendication 1 ou 2, dans lequel les une ou plusieurs de la longueur d'onde, fréquence ou intensité du signal lumineux peuvent être modifiées.

4. Système de traitement des déchets selon une quelconque revendication précédente, dans lequel le signal lumineux est transmis à travers des fenêtres optiques opposées (46) dans un chemin de fluide à travers lequel circule le mélange de prétraitement.

5. Système de traitement des déchets selon une quelconque revendication précédente, dans lequel le signal lumineux est reçu à une photodiode (38) après avoir traversé le mélange de prétraitement.

6. Système de traitement des déchets selon une quelconque revendication précédente, comprenant également un filtre optique disposé entre la source lumineuse et le mélange de prétraitement.

7. Système de traitement des déchets selon une quelconque revendication précédente, dans lequel les une ou plusieurs propriétés comportent une concentration de prétraitement urinaire dans le mélange de prétraitement et une qualité de mélange du mélange de prétraitement.

8. Système de traitement des déchets selon une quelconque revendication précédente, comprenant également un dispositif de commande (40) pour comparer les une ou plusieurs propriétés à une plage présélectionnée.

9. Procédé de surveillance d'un flux d'un mélange de prétraitement d'un système de traitement des déchets, comprenant :
le mélange d'un volume de prétraitement urinaire avec un volume d'eau à une pompe doseuse (22) définissant un mélange de prétraitement ;
le fait de diriger le mélange de prétraitement le long d'un chemin de fluide vers un réservoir de stockage (16) ;
la mesure d'une ou de plusieurs propriétés du mélange de prétraitement via un capteur optique (30) disposé le long du chemin de fluide.

10. Procédé selon la revendication 9, dans lequel le capteur optique :
transmet un signal lumineux à travers le chemin de fluide via une source lumineuse (34), et
reçoit le signal lumineux à une photodiode (38) après avoir traversé le mélange de prétraitement.

11. Procédé selon la revendication 10, dans lequel la source lumineuse est une source lumineuse DEL ; et/ou comprenant également la variation d'une ou de plusieurs d'une longueur d'onde, fréquence ou intensité du signal lumineux ; et/ou comprenant également la transmission du signal lumineux à travers des fenêtres optiques opposées (46) dans le chemin de fluide.

12. Procédé selon la revendication 10 ou 11, comprenant également la transmission du signal lumineux à travers un filtre disposé entre la source lumineuse et le chemin de fluide.

13. Procédé selon l'une quelconque des revendications 9 à 12, comprenant également la comparaison des une ou plusieurs propriétés à une plage présélectionnée.

14. Procédé selon la revendication 13, comprenant également le déclenchement d'une alarme lorsque les une ou plusieurs propriétés sont en dehors de la plage présélectionnée.

15. Procédé selon l'une quelconque des revendications 9 à 14, dans lequel les une ou plusieurs propriétés comportent une concentration de prétraitement urinaire dans le mélange de prétraitement et une qualité de mélange du mélange de prétraitement.
